# EUROPÄISCHE PATENTSCHRIFT

(11) **EP 2 635 244 B1**
(45) Veröffentlichungstag und Bekanntmachung des Hinweises auf die Patenterteilung: **30.03.2016**
(21) Anmeldenummer: 11785360.6
(22) Anmeldetag: 01.11.2011
(51) Int. Cl.: A61F 13/00

(54) **WUNDAUFLAGE MIT EINER BIOCELLULOSESCHICHT, DIE BAKTERIEN ADSORBIEREND AUSGERÜSTET IST**
WOUND DRESSING COMPRISING A BIOCELLULOSE LAYER HAVING A BACTERIA-ADSORBING DESIGN
PANSEMENT POUR PLAIE COMPRENANT UNE COUCHE DE BIOCELLULOSE ET ÉQUIPÉ POUR ADSORBER DES BACTÉRIES

(30) Priorität: 03.11.2010 DE 102010050311
(43) Veröffentlichungstag der Anmeldung: 11.09.2013
(73) Patentinhaber: BSN Medical GmbH, 20253 Hamburg (DE)
(72) Erfinder: RAMMINGER, Ralf, 22941 Bargteheide (DE); ANDREWS, Hugh, 21244 Buchholz (DE); SCHÜTZ, Patrick, 21075 Hamburg (DE)
(74) Vertreter: UEXKÜLL & STOLBERG
(86) Internationale Anmeldenummer: PCT/EP2011/069191
(87) Internationale Veröffentlichungsnummer: WO 2012/059488

(56) Entgegenhaltungen:
- EP-A1- 1 438 975
- EP-A1- 1 473 047
- WO-A1-2006/062470
- US-A- 4 588 400
- US-A- 4 788 146
- US-A1- 2006 163 149
- US-A1- 2008 177 214

## Beschreibung

Die vorliegende Erfindung betrifft eine Wundauflage mit mindestens einer Schicht aus mikrobiell hergestellter Cellulose (Biocelluloseschicht). Die Wundauflage ist so ausgerüstet, dass sie Bakterien adsorbiert.

Wundauflagen für die Heilung von Verbrennungen, Hautverletzungen oder schlecht heilenden Wunden sind bekannt. So beschreiben die US 4,588,400 A, die US 4,655,758 A und die US 4,788,146 A Materialien für derartige Anwendungen, die auf der Basis von mikrobiell hergestellter Cellulose hergestellt werden. Diese Dokumente beschreiben ferner, dass die Biocelluloseschicht mit bakteriostatischen Mitteln wie Silbernitrat oder Benzalkoniumchlorid getränkt werden kann. Verfahren zur Herstellung von Biocellulose sind ferner in der WO 86/02095 A1 und der WO 2004/050986 A beschrieben. Auch die EP 1 356 831 A beschreibt die Anwendung von Biocellulose bei der Wundbehandlung.

Gemäß der WO 2005/009276 A kann Biocellulose mit Polyhexamethylenbiguanid-Hydrochlorid antimikrobiell ausgerüstet sein. Die entsprechenden Wundauflagen sollen Feuchtigkeit abgeben und sind dementsprechend durch einen sehr hohen Wassergehalt und einen entsprechend geringen Cellulosegehalt gekennzeichnet. Die Anwendung der Wundauflagen der WO 2005/009276 A erfolgt speziell bei chronischen Wunden. Bei einer Brandwunde kann zuviel Feuchtigkeit jedoch kontraindiziert sein.

EP 1 473 047 A1 betrifft eine Wundauflage, die mikrobielle Cellulose umfasst, zur Behandlung von bestimmten Arten von chronischen Wunden, einschließlich Druckgeschwüren und Venen- und diabetischen Geschwüren.

US 2006/163149 A1 offenbart ein Produkt für Absorptionszwecke, das eine wasserunlösliche Trägermatrix einschließt, wobei die Trägermatrix mit einer hydrophoben Gruppe substituiert ist, die wiederum mit einer positiv geladenen Gruppe verbunden ist.

US 2008/177214 A1 beschreibt eine Wundauflage, die ein hydrophobes Gewebe, das der Wunde zugewandt ist, und das in der Lage ist, unerwünschte Mikroorganismen zu binden, und eine Feuchtigkeit enthaltende Matrix wie ein Gel oder einen Schaum kombiniert.

Die EP 1 438 975 A offenbart ein Feuchtigkeit abgebendes amorphes Hydrogel auf Basis mikrobiell veränderter Cellulose zur Aufbringung auf Wunden. Neben den erwähnten Nachteilen einer hohen Feuchtigkeit (bei einer Brandwunde kontraindiziert) kann durch ein solches Gel die Infizierung der Wunde gefördert werden.

Das Produkt Cuticell^{®} Epigraft der BSN medical GmbH, Hamburg, Bundesrepublik Deutschland, besteht aus einer reinen Cellulosemembran, die vom Mikroorganismus *Acetobacter xylinum* gebildet wird. Das Produkt ist transparent und erlaubt somit, den Wundheilungsverlauf während der Behandlung zu beobachten, ohne dass die Wundauflage zerstört werden muss. Solche Wundauflagen auf Basis von Biocellulose sind für die Behandlung von Spalthautentnahmestellen, Verbrennungen, Hautrissen, Wunden und Hautläsionen (wie Hautabschürfungen, Schnittwunden und kleinen chirurgischen Wunden) geeignet. Allerdings sind handelsübliche Wundauflagen auf der Basis von Biocellulose dünn und somit bei der Anwendung empfindlich. Bei unsachgemäßer Anwendung kann es leicht zu einer Verkeimung kommen. Infizierte Wunden können also nicht mit Wundauflagen auf Basis von Biocellulose behandelt werden. Somit besteht ein Bedarf an Wundauflagen, die auch bei infizierten Wunden sowie chronischen Wunden mit Exsudat angewendet werden können.

Darüber hinaus sind Bakterien adsorbierende Zusammensetzungen bekannt. So beschreibt die EP 0 162 026 A, dass Bakterien adsorbierende Zusammensetzungen zur Behandlung von äußeren Infektionen verwendet werden können, was zu einer besseren Entfernung von Bakterien und anderen Mikroorganismen führt. Die Zusammensetzungen enthalten ein hydrophiles Material, wie Baumwolle, die durch Behandlung mit beispielsweise Dialkylcarbamoylchlorid hydrophobiert ist.

Außerdem offenbart die WO 2006/062470 A eine Wundauflage, die eine solche Mikroorganismen bindende hydrophobe Schicht auf der der Wunde zugewandten Seite, darüber eine absorbierende hydrophile Schicht und als Deckschicht eine vorzugsweise transparente Polyurethanschicht aufweist. Die Hauptaufgabe solcher Produkte liegt bei der Adsorption von Bakterien, d.h. der Einsatzbereich derartiger Produkte ist beschränkt und sie müssen stets mit einer weiteren Wundauflage kombiniert werden. Derartige Produkte sind also besonders für die kurzzeitige Wundabdeckung geeignet.

Auch in der DE 196 31 421 A geht es um hydrophobierte Trägermaterialien, die Bakterien adsorbieren können. Das Produkt Cutisorb^{®} Sorbact^{®} der BSN medical GmbH, Hamburg, Bundesrepublik Deutschland, weist beispielsweise eine Schicht aus hydrophobiertem Acetat- oder Baumwollgewebe auf.

DE 101 35 676 A offenbart, dass (gegebenenfalls mikrobiell produzierte) Cellulose mit Chitosan, Hyaluronsäure oder Derivaten oder Mischungen davon dotiert werden kann. Der dotierte Film wird unter anderem als physiologisches Diaphragma, künstliche Blutbahn und/oder Infusions- oder Dialyseschlauch eingesetzt. In solchen Anwendungen ist es ausgeschlossen, dass Protein absorbiert werden, weshalb die Filme gemäß DE 101 35 676 A nicht Bakterien adsorbieren.

Der vorliegenden Erfindung hat somit die Aufgabe zugrunde gelegen, den Einsatzbereich von Wundauflagen, insbesondere Wundauflagen auf Basis von Biocellulose, zu erweitern und zu verbessern. Durch die erfindungsgemäßen Wundauflagen soll ein besonderes Wundklima geschaffen werden, das von äußeren Einflüssen abgeschottet ist. Die Nanostruktur der Biocellulose eignet sich dabei gut zur Anlagerung neugebildeter Zellen.

Es hat sich nun überraschend herausgestellt, dass diese Aufgabe durch eine Wundauflage gemäß Anspruch 1 gelöst wird, die über mindestens eine Schicht aus mikrobiell gebildeter Cellulose (im Folgenden Biocelluloseschicht) verfügt, wobei die Wundauflage so ausgerüstet ist, dass Bakterien adsorbiert werden. Die Biocelluloseschicht besitzt eine Dicke von 0,08 bis 1,5 mm.

In einer bevorzugten Ausführungsform ist die Biocelluloseschicht durch die folgenden Parameter gekennzeichnet:
- eine Dicke von 0,10-0,25 mm,
- eine Wasserdampfdurchlässigkeit von mehr als 300 g/m² * 24 h, vorzugsweise von mehr als 500 g/m² * 24 h, insbesondere 1000 - 3000 g/m² * 24h bei 38°C, und/oder
- ein Flächengewicht von 7 bis 75 g/m², vorzugsweise 9-50 g/m², insbesondere 10-40 g/m², insbesondere 11 - 22 g/m².

Vorteilhafterweise handelt es sich bei der Biocellulose um eine gemäß einem statischen Verfahren hergestellte Biocellulose. Diese weist eine vorteilhafte dreidimensionale Mehrschichtstruktur auf. Im Gegensatz dazu sind Biocellulosetypen nicht bevorzugt, die in einem Rührtank-Bioreaktor gemäß der Lehre der US 5,846,213 A1 hergestellt werden.

Gemäß einer Ausführungsform der Erfindung ist die Wundauflage dadurch gekennzeichnet, dass die Bakterien adsorbierende Ausrüstung darin besteht, dass die Biocelluloseschicht selbst hydrophob ausgerüstet ist. Diese Hydrophobausrüstung kann eine Behandlung mit Dialkylcarbamoylchlorid und/oder Alken-Keten-Dimer sein, wie sie im Stand der Technik bekannt ist.

Bei bevorzugten Verfahren für die Hydrophobausrüstung der Biocelluloseschicht wird eine solche Schicht hydrophob ausgerüstet, deren Biocelluloseanteil größer als etwa 5 Gew.-% ist, vorzugsweise größer als 20 Gew.-%, bevorzugter größer als 60 Gew.-%, insbesondere größer als 80 Gew.-%, wie größer als 90 Gew.-%, größer als 95 Gew.-% oder sogar größer als 97 Gew.-%, wie etwa 99 Gew.-%.

Erfindungsgemäß besteht die Bakterien adsorbierende Ausrüstung der Wundauflage darin, dass die Wundauflage zusätzlich zu der Biocelluloseschicht mindestens eine hydrophobe Schicht auf derjenigen Seite der Biocelluloseschicht aufweist, die der Wunde zugewandt ist.

Diese zusätzliche hydrophobe Schicht kann Celluloseacetatgewebe, Viskosegewebe, Baumwollgewebe oder einen Mischgewebe enthalten, wobei als Mischgewebe Schmelzklebfasern bevorzugt sind. Besonders bevorzugt sind hydrophobe Schichten auf Basis von Celluloseacetatgewebe und Baumwollgewebe, insbesondere Celluloseacetatgewebe. Die Hydrophobierung der hydrophoben Schicht besteht in einer Behandlung mit Dialkylcarbamoylchlorid und/oder Alken-Keten-Dimer. In einer bevorzugten Ausführungsform sind die hydrophobe Schicht und die Biocelluloseschicht in einem Herstellungsprozess zusammengewachsen. Alternativ ist es möglich, dass sich zwischen der hydrophoben Schicht und der Biocelluloseschicht eine Klebeschicht befindet, oder dass die zwei Schichten durch ein Kaschierverfahren zusammengefügt worden sind.

Für den Fall, dass das Basismaterial, welches der Herstellung der hydrophoben Schicht zugrunde liegt, ursprünglich hydrophil ist, wie dies beispielsweise bei Cellulose und Viskose der Fall ist, ist dieses Basismaterial mit Hilfe einer Beschichtung behandelt, um die hydrophobe Schicht zu ergeben.

Gemäß einer Ausführungsform ist bei der erfindungsgemäßen Wundauflage sowohl die Biocelluloseschicht hydrophob ausgerüstet als auch eine hydrophobe Schicht auf derjenigen Seite der Biocelluloseschicht vorhanden, die der Wunde zugewandt ist.

Darüber hinaus kann sich auf der Seite der Biocelluloseschicht, die der Wunde abgewandt ist, eine Deckschicht befinden. In einer bevorzugten Ausführungsform befindet sich jedoch auf der Seite der Biocelluloseschicht, die der Wunde abgewandt ist, keine Deckschicht. Stattdessen wird die Wunde normal bandagiert.

Die Herstellung der Biocellulose durch Bakterien basiert auf der Ausscheidung von nanofeinen Cellulosefilamenten durch die Bakterien. Dieser Prozess findet kontrolliert unter bestimmten klimatischen Bedingungen in einer Nährlösung statt. Es handelt sich somit um einen biotechnologischen Prozess. Die Bakterien scheiden nach und nach so viel Cellulose aus, dass sich ein Gelkissen aus Biocellulose bildet. Durch Formgebung oder Einbringen in das noch im Wachsen befindliche Gelkissen aus Biocellulose kann das Endprodukt beeinflusst werden. Beispielsweise ist es also möglich, die zusätzliche hydrophobe Schicht und die Biocelluloseschicht durch Einwachsen der Bakterien während des biotechnologischen Herstellprozesses miteinander zu verbinden.

Die Biocellulose kann so gewachsen sein, dass sie offene Strukturen hat, um einerseits durch direkten Kontakt die Wundheilung zu fördern und andererseits die Wundflüssigkeit der infizierten Wunde in die Wundauflage hinein zutransportieren, wobei sich die Wundflüssigkeit mit den darin enthaltenen infektiösen Bakterien anlagert.

In allen Ausführungsformen der Erfindung ist es bevorzugt, dass die Wundauflage mit einer oder mehreren antimikrobiellen Substanz(en) antimikrobiell ausgerüstet ist. Diese Ausrüstung kann in einer Tränkung der Biocelluloseschicht bestehen, oder in einer Tränkung der hydrophoben Schicht, oder in einer Tränkung der Biocellulose- und der hydrophoben Schicht. Alternativ kann die fertige Wundauflage mit allen enthaltenen Schichten antimikrobiell ausgerüstet sein.

Die Erfindung eignet sich insbesondere für präventive Anwendung bei Spalthautentnahmen oder bei Brandwunden, insbesondere bei diesen Wunden, wenn sie bereits infiziert sind.

Spalthaut eignet sich insbesondere zum Decken großer Flächen, z.B. nach Verbrennungen, zumal sich die Spenderstellen in kurzer Zeit regenerieren können. Die Entnahme erfolgt üblicherweise an ebenen Hautarealen, bevorzugt am Oberschenkel, Rücken, Gesäß oder Bauch. Bei der Gewinnung von Spalthaut werden, nach Unterspritzung mit Lokalanästhetikum, die obersten Epithelschichten flächenhaft mit dem Skalpell oder Dermatom abgetragen.

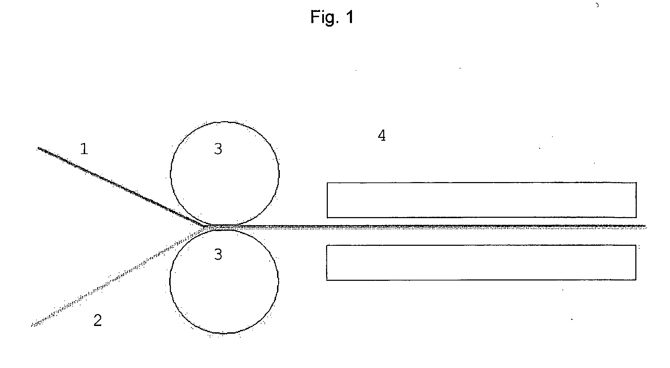

Darüber hinaus betrifft die Erfindung auch ein Verfahren zur Herstellung der erfindungsgemäßen Wundauflagen. Bei einem bevorzugten Verfahren, das in Fig. 1 dargestellt ist, wird die Biocelluloseschicht im feuchten Zustand bereitgestellt. Die Bakterien adsorbierende Ausrüstung erfolgt durch Kalandrieren. Unmittelbar im Anschluss an das Kalandrieren wird die Wundauflage ofengetrocknet. Dabei bezeichnet in Figur 1
(1) die Biocelluloseschicht im feuchten Zustand,
(2) die hydrophobe Schicht,
(3) die Kalandrierwalzen und
(4) den Ofen zum Trocknen.

In einer ersten Ausführungsform umfasst die erfindungsgemäße Wundauflage auf der Wundseite ein (vorzugsweise offenmaschiges) Acetatgewirk oder Baumwollgewebe mit einem Flächengewicht von 60 bis 200 g/m², vorzugsweise 80 bis 150 g/m², insbesondere 90 bis 130 g/m², wie 110 g/m², ausgerüstet mit einer mikrobiell adhäsiven Substanz, wie zum Beispiel DAAC, in Verbindung mit einer oberseitigen Schicht Biocellulose mit einem Flächengewicht von 3 bis 100 g/m², vorzugsweise 5 bis 80 g/m², insbesondere 10 bis 40 g/m².

In einer weiteren Ausführungsform umfasst die erfindungsgemäße Wundauflage ein Vlies auf Cellulosebasis (Viskose, Baumwolle oder als Gemisch mit nicht cellulosischen Fasern) mit einem Flächengewicht von 5 bis 120 g/m², vorzugsweise 10 bis 100 g/m², insbesondere 20 bis 60 g/m², ebenfalls ausgerüstet mit einer mikrobiell adhäsiven Substanz, und einer oberseitigen Biocelluloseschicht.

In einer dritten Ausführungsform umfasst die erfindungsgemäße Wundauflage das wie oben beschriebene Acetatgewebe, welches von Biocellulose umschlossen ist, sodass das Gesamtflächengewicht der mikrobiellen Cellulose einschließlich des Acetatgewebes etwa 10 bis 150 g/m², vorzugsweise 20 bis 120 g/m², insbesondere 30 bis 80 g/m² beträgt.

## Patentansprüche

1. Wundauflage mit mindestens einer Schicht aus mikrobiell hergestellter Cellulose (Biocelluloseschicht), wobei
(i) die Wundauflage so ausgerüstet ist, dass Bakterien adsorbiert werden, und
(ii) die Biocelluloseschicht der Wundauflage eine Dicke von 0,08 bis 1,5 mm besitzt,
wobei die Bakterien adsorbierende Ausrüstung darin besteht, dass die Wundauflage zusätzlich zu der Biocelluloseschicht mindestens eine hydrophobe Schicht auf der Seite der Biocelluloseschicht aufweist, die der Wunde zugewandt ist, und
wobei die hydrophobe Schicht mit Dialkylcarbamoylchlorid und/oder Alken-Keten-Dimer behandelt worden ist.

2. Wundauflage nach Anspruch 1, **dadurch gekennzeichnet, dass** die Biocelluloseschicht eine Dicke von 0,10 - 0,25 mm besitzt.

3. Wundauflage nach einem der vorhergehenden Ansprüche, **dadurch gekennzeichnet, dass** die Biocelluloseschicht ein Flächengewicht von 7 bis 75 g/m² besitzt.

4. Wundauflage nach Anspruch 3, **dadurch gekennzeichnet, dass** die Biocelluloseschicht ein Flächengewicht von 9 - 50 g/m² besitzt.

5. Wundauflage nach Anspruch 4, **dadurch gekennzeichnet, dass** die Biocelluloseschicht ein Flächengewicht von 10 - 40 g/m² besitzt.

6. Wundauflage nach Anspruch 5, **dadurch gekennzeichnet, dass** die Biocelluloseschicht ein Flächengewicht von 11 - 22 g/m² besitzt.

7. Wundauflage nach einem der vorhergehenden Ansprüche, **dadurch gekennzeichnet, dass** die Bakterien adsorbierende Ausrüstung darin besteht, dass die Biocelluloseschicht hydrophob ausgerüstet ist.

8. Wundauflage nach Anspruch 7, **dadurch gekennzeichnet, dass** die Hydrophobausrüstung der Biocelluloseschicht eine Behandlung mit Dialkylcarbamoylchlorid und/oder Alken-Keten-Dimer ist.

9. Wundauflage nach Anspruch 1, **dadurch gekennzeichnet, dass** die hydrophobe Schicht Celluloseacetatgewebe, Viskosegewebe, Baumwollgewebe oder ein Mischgewebe ist.

10. Wundauflage nach Anspruch 9, **dadurch gekennzeichnet, dass** das Mischgewebe Schmelzklebefasern umfasst.

11. Wundauflage nach einem der vorhergehenden Ansprüche, **dadurch gekennzeichnet, dass** die hydrophobe Schicht und die Biocelluloseschicht durch Einwachsen der Bakterien während des biotechnologischen Herstellprozesses miteinander verbunden sind.

12. Wundauflage gemäß einem der Ansprüche 1 bis 11 zur Verwendung in einem Verfahren zur präventiven Anwendung bei Spalthautentnahme oder bei Brandwunden, insbesondere bei diesen Wunden, wenn sie bereits infiziert sind.

## Claims

1. Wound dressing having at least one layer of microbially produced cellulose (biocellulose layer), wherein
(i) the wound dressing is finished in such a way that bacteria are adsorbed, and
(i) the biocellulose layer of the wound dressing has a thickness of 0.08 to 1.5 mm,
wherein the bacteria-adsorbent finish consists in the wound dressing having, in addition to the biocellulose layer, at least one hydrophobic layer on that side of the biocellulose layer which faces the wound, and
wherein the hydrophobic layer has been treated with dialkyl carbamoyl chloride and/or alkene ketene dimer.

2. Wound dressing according to Claim 1, **characterized in that** the biocellulose layer has a thickness of 0.10 - 0.25 mm.

3. Wound dressing according to one of the preceding claims, **characterized in that** the biocellulose layer has a weight per unit area of 7 to 75 g/m².

4. Wound dressing according to Claim 3, **characterized in that** the biocellulose layer has a weight per unit area of 9 - 50 g/m².

5. Wound dressing according to Claim 4, **characterized in that** the biocellulose layer has a weight per unit area of 10 - 40 g/m².

6. Wound dressing according to Claim 5, **characterized in that** the biocellulose layer has a weight per unit area of 11 - 22 g/m².

7. Wound dressing according to one of the preceding claims, **characterized in that** the bacteria-adsorbent finish consists in the biocellulose layer being finished hydrophobically.

8. Wound dressing according to Claim 7, **characterized in that** the hydrophobic finishing of the biocellulose layer is a treatment with dialkyl carbamoyl chloride and/or alkene ketene dimer.

9. Wound dressing according to Claim 1, **characterized in that** the hydrophobic layer is cellulose acetate fabric, viscose fabric, cotton fabric or a mixed fabric.

10. Wound dressing according to Claim 9, **characterized in that** the mixed fabric comprises hot-melt fibres.

11. Wound dressing according to one of the preceding claims, **characterized in that** the hydrophobic layer and the biocellulose layer are joined to each other as a result of ingrowth of the bacteria during the biotechnological production process.

12. Wound dressing according to one of Claims 1 to 11 for use in a method for preventive application in split skin removal or in burns, in particular in these wounds when they are already infected.

## Revendications

1. Pansement comportant au moins une couche d'une cellulose fabriquée par voie microbienne (couche de biocellulose), dans lequel
(i) le pansement est apprêté de façon que des bactéries soient adsorbées, et
(ii) la couche de biocellulose du pansement a une épaisseur de 0,08 à 1,5 mm,
l'apprêt adsorbant les bactéries consistant en ce que le pansement comprend, en plus de la couche de biocellulose, au moins une couche hydrophobe sur le côté de la couche de biocellulose qui est dirigée vers la plaie, et
la couche hydrophobe ayant été traitée par un chlorure de dialkylcarbamoyle et/ou un dimère alcène-cétène.

2. Pansement selon la revendication 1, **caractérisé en ce que** la couche de biocellulose présente une épaisseur de 0,10 à 0,25 mm.

3. Pansement selon l'une des revendications précédentes, **caractérisé en ce que** la couche de biocellulose présente une masse surfacique de 7 à 75 g/m².

4. Pansement selon la revendication 3, **caractérisé en ce que** la couche de biocellulose présente une masse surfacique de 9 à 50 g/m².

5. Pansement selon la revendication 4, **caractérisé en ce que** la couche de biocellulose présente une masse surfacique de 10 à 40 g/m².

6. Pansement selon la revendication 5, **caractérisé en ce que** la couche de biocellulose présente une masse surfacique de 11 à 22 g/m².

7. Pansement selon l'une des revendications précédentes, **caractérisé en ce que** l'apprêt adsorbant les bactéries consiste en un apprêt hydrophobe de la couche de biocellulose.

8. Pansement selon la revendication 7, **caractérisé en ce que** l'apprêt hydrophobe de la couche de biocellulose est un traitement avec un chlorure de dialkylcarbamoyle et/ou un dimère alcène-cétène.

9. Pansement selon la revendication 1, **caractérisé en ce que** la couche hydrophobe est un tissu d'acétate de cellulose, un tissu de viscose, un tissu de coton ou un tissu mélangé.

10. Pansement selon la revendication 9, **caractérisé en ce que** le tissu mélangé comprend des fibres adhésives fusibles.

11. Pansement selon l'une des revendications précédentes, **caractérisé en ce que** la couche hydrophobe et la couche de biocellulose sont assemblées l'une à l'autre par ancrage des bactéries pendant l'opération de fabrication biotechnologique.

12. Pansement selon l'une des revendications 1 à 11 pour une utilisation dans un procédé destiné à une utilisation préventive lors du prélèvement de peau mince ou dans le cas de brûlures, notamment dans le cas des plaies quand elles sont déjà infectées.
